# EUROPEAN PATENT SPECIFICATION

(11) **EP 1 203 568 B1**
(45) Date of publication and mention of the grant of the patent: **11.02.2004**
(21) Application number: 01125661.7
(22) Date of filing: 26.10.2001
(51) Int. Cl.: A61F 2/08

(54) **Tendon tensioning device**
Sehnenspannvorrichtung
Dispositif pour tensioner un tendon

(30) Priority: 27.10.2000 IT BO000629
(43) Date of publication of application: 08.05.2002
(73) Proprietor: Tecnologie Dinamiche S.A.S. di Rossi Vincenzo & C., 40132 Bologna (IT)
(72) Inventor: Rossi, Vincenzo, 40012 Calderara di Reno (IT)
(74) Representative: Cerbaro, Elena, Dr.

(56) References cited:
- EP-A- 0 379 789
- US-A- 5 713 897
- US-A- 6 001 106

## Description

The present invention relates to a tendon tensioning device.

As is known from document US 6001106, in orthopedics, tendons are tensioned before being implanted into the patient's body. At present, this is done manually by an assistant before the tendon is implanted, which obviously fails to ensure the degree of precision called for in this type of operation. More specifically, the tendon may be undertensioned and so be shorter than necessary, or may be overtensioned so that the component tissues exceed the viscous point at which yield occurs.

It is an object of the present invention to provide a device designed to prevent undue stress of the component tissues of tendons for implantation. The device is also designed to implement a tendon tensioning method, which is a further object of the present invention.

A non-limiting embodiment of the present invention will be described by way of example with reference to the accompanying drawings, in which:
Figure 1 shows a side view of a device in accordance with the present invention;
Figure 2 shows a plan view of the Figure 1 device;
Figure 3 shows a cross section A-A of the Figure 1 and 2 device.

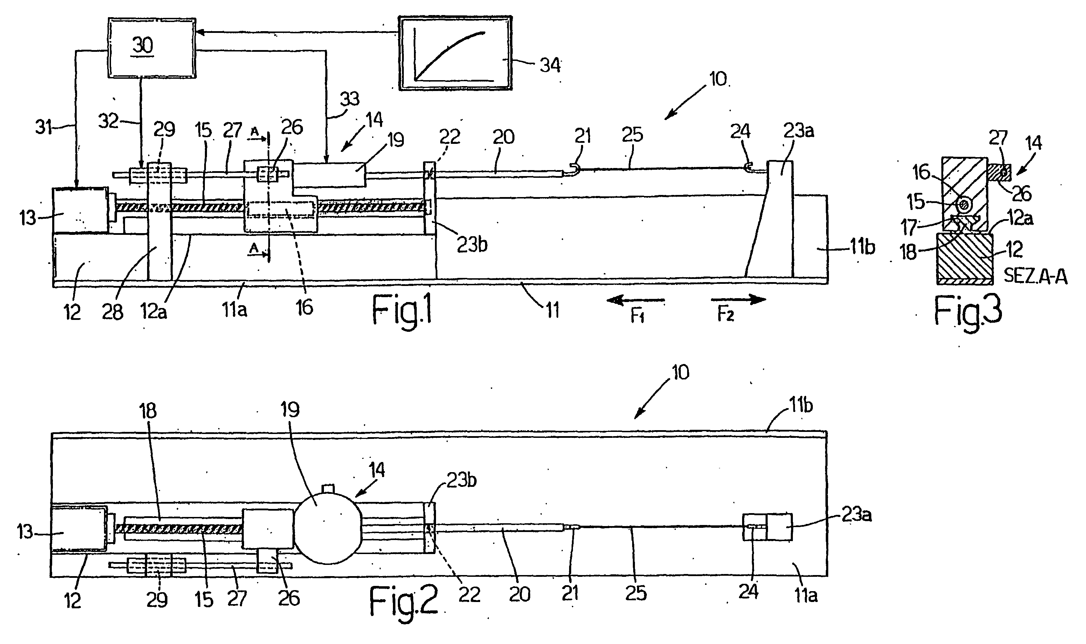

Number 10 in Figures 1 and 2 indicates as a whole a tendon tensioning device in accordance with the present invention.

Tensioning device 10 comprises a support 11, in turn comprising a base plate 11a and a side 11b.

A supporting block 12 rests firmly on base plate 11a, and supports, as described in detail later on, an electric motor 13 and a slide 14. Electric motor 13 simply rests on, and is fixed to, the top surface 12a of block 12; a screw 15, rotated by motor 13, engages a feed nut 16 forming part of slide 14; and slide 14 also comprises a "dovetail" cavity 17 engaged by a member 18 projecting from top surface 12a of block 12. In other words, slide 14 and block 12 are joined by a "dovetail" connection permitting translation of slide 14 in either of the two directions indicated by arrows F1 and F2.

Therefore, by rotating screw 15 in one direction or the other by means of electric motor 13, slide 14 can be moved in directions F1 and F2.

On the opposite side to motor 13, slide 14 is fitted with a load cell 19, from which projects a pull rod 20 fitted with a hook 21. To ensure it moves parallel to screw 15 without flexing, rod 20 is guided by a seat 22 formed in a supporting member 23b projecting from surface 12a of block 12.

A direct bearing 23a, fitted with a hook 24, projects upwards from base plate 11a.

A tendon 25, to be treated as described in detail below, is stretched between hook 21 integral with rod 20, and hook 24 integral with bearing 23a.

To control stretching of tendon 25, an arm 26 projects from slide 14 and is integral with a graduated rod 27; and a direct bearing 28, extending upwards from base plate 11a, supports detecting means 29 for detecting displacement of graduated rod 27, which, as stated, is integral with slide 14.

An electronic central control unit 30, connected electrically to motor 13, load cell 19, and means 29 by respective electric lines 31, 33 and 32, provides for electronically processing detected data to regulate the pull exerted by means of motor 13 as described in detail with reference to the process performed on tendon 25 by device 10. Finally, device 10 is completed by a screen 34 for displaying graphs and bar graphs of any type.

To better regulate the displacement of slide 14, electric motor 13 may be an electric, e.g. 512 steps/revolution, step motor.

Though Figures 1-3 show a straightforward screw 15/feed nut 16 coupling to reduce friction and better regulate the position of slide 14, a ball bearing coupling may also be used.

As opposed to the "dovetail" connection between slide 14 and block 12, any type of connection, e.g. rails, may be used without departing from the scope of the present invention.

Device 10 provides for performing the following process on tendon 25:
(a) - zeroing the length of tendon 25 and determining the point at which tension on the tendon begins to increase alongside an increase in applied load;
(b) - setting a target value of the maximum load to be applied to tendon 25;
(c) - increasing the applied-load value towards the maximum value set at step (b) until a fall in tension applied to tendon 25 is detected; at this step, slide 14 is drawn by motor 13 in the direction of arrow F1;
(d) - determining the viscous crossover point of the component tissues of tendon 25, and the load at which crossover occurs; and
(e) - returning to a load value, on tendon 25, below the one corresponding to the viscous point, and at which elastic performance of the tissues is restored; at this step, slide 14 is moved by motor 13 in the direction of arrow F2.

It is also extremely important that the assistant determine the difference between the load value at which viscous crossover of the tissues occurs (see step (d)), and the load value at which elastic performance of the tissues is restored (see step (e)); which difference provides the assistant with fairly accurate data as to the possible extension of tendon 25 once implanted into the patient's body.

Another vital quantity is the stretch to which tendon 25 is subjected.

For which purpose, as stated, means 29 are provided, which, together with graduated rod 27, determine the position of slide 14.

All the significant data detected and processed by central control unit 30 are displayed on screen 34.

More specifically, screen 34 displays graphs showing, along the x axis, the load values induced in tendon 25 by displacement of slide 14 in directions F1 and F2, and, along the y axis, the actual tension values detected in tendon 25 and measured by load cell 19. The graph may also show when the near-yield viscous point is reached, and quantify the difference between the load value at which viscous crossover of the tissues occurs (see step (d)), and the load value at which elastic performance of the tissues is restored (see step (e)).

Screen 34 may also display a coloured bar graph showing stretch of tendon 25 and the data detected by means 29.

## Claims

1. A tensioning device (10) for tensioning a tendon (25) comprising:
- a slide (14) and a supporting member (23a), said tendon (25) being retained by retaining means (21, 24) integral with said slide (14) and said supporting member (23a) respectively;
- detecting means (19) for detecting the actual tension induced in said tendon (25); the tensioning device (10) being **characterized by**
- a motor (13) connected mechanically to said slide (14) and such as to move said slide (14) in two directions (F1, F2).

2. A device (10) as claimed in Claim 1, wherein said motor (13) is an electric motor (13), in particular an electric step motor (13).

3. A device (10) as claimed in Claim 2, wherein said electric step motor (13) is a 512 steps/revolution electric motor (13).

4. A device (10) as claimed in any one of the foregoing Claims, and also comprising means (27, 29) for determining the position of said slide (14) and therefore the stretch of said tendon (25).

5. A device (10) as claimed in any one of the foregoing Claims, and also comprising a screen (34).

6. A device (10) as claimed in Claim 5, wherein said screen (34) displays graphs showing, along the x axis, the load values induced in said tendon (25) by displacement of said slide (14), and, along the y axis, the actual tension values detected in the tendon (25) and measured by a load cell (19).

7. A device (10) as claimed in Claim 5, wherein said graphs show when the near-yield viscous point of said tendon (25) is reached, and quantify the difference between the load value at which viscous crossover of the tissues of the tendon (25) occurs, and the load value at which elastic performance of the tissues of the tendon (25) is restored.

8. A device (10) as claimed in any one of the foregoing Claims, wherein all the operations are controlled by an electronic central control unit (30).

9. A method of tensioning a tendon before being implanted into the body, **characterized by** comprising the steps of:
(a) - zeroing the length of the tendon and determining the point at which tension on the tendon begins to increase alongside an increase in applied load;
(b) - setting a target value of the maximum load to be applied to the tendon;
(c) - increasing the applied-load value towards the maximum value set at step (b) until a fall in tension applied to the tendon is detected;
(d) - determining the viscous crossover point of the component tissues of the tendon, and the load at which crossover occurs; and
(e) - returning to a load value, on the tendon, below the one corresponding to the viscous point, and at which elastic performance of the tissues is restored.

10. A method as claimed in Claim 9, wherein the difference between the load value as per step (d) and the load value as per step (e) is determined.

## Patentansprüche

1. Spannvorrichtung (10) für das Spannen einer Sehne (25), umfassend:
einen Schlitten (14) und ein Trägerungselement (23a), wobei die Sehne (25) durch Haltemittel (21, 24), die mit dem Schlitten (14) bzw. dem Trägerungselement (23a) eine Einheit bilden, gehalten wird;
Erkennungsmittel (19) zum Erkennen der tatsächlichen Zugspannung, der die Sehne (25) unterworfen ist;
wobei die Spannvorrichtung (10) **gekennzeichnet ist durch** einen Motor (13), der mechanisch mit dem Schlitten (14) dergestalt verbunden ist, dass er den Schlitten (14) in zwei Richtungen (F1, F2) bewegen kann.

2. Vorrichtung (10) nach Anspruch 1, wobei der Motor (13) ein Elektromotor (13) ist, insbesondere ein elektrischer Schrittmotor (13).

3. Vorrichtung (10) nach Anspruch 2, wobei der elektrische Schrittmotor (13) ein Elektromotor (13) mit 512 Schritten/Umdrehung ist.

4. Vorrichtung (10) nach einem der vorangegangenen Ansprüche, die außerdem Mittel (27, 29) für das Feststellen der Position des Schlittens (14) und somit der Dehnung der Sehne (25) umfasst.

5. Vorrichtung (10) nach einem der vorangegangenen Ansprüche, die außerdem einen Bildschirm (34) umfasst.

6. Vorrichtung (10) nach Anspruch 5, wobei der Bildschirm (34) Kurvenschaubilder anzeigt, die entlang der x-Achse die Belastungswerte anzeigen, die in der Sehne (25) durch Verschiebung des Schlittens (14) entstehen, und die entlang der y-Achse die tatsächlichen Zugspannungswerte anzeigen, die in der Sehne (25) erkannt und durch die Kraftmessdose 19 gemessen werden.

7. Vorrichtung (10) nach Anspruch 5, wobei die Kurvenschaubilder angezeigt werden, wenn der dehngrenzennahe Viskositätspunkt der Sehne (25) erreicht ist, und wobei die Kurvenschaubilder die Differenz zwischen dem Belastungswert, bei dem sich die Viskositätsüberschneidung der Gewebe der Sehne (25) vollzieht, und dem Belastungswert, bei dem die Elastizität der Gewebe der Sehne (25) wiederhergestellt ist, quantifizieren.

8. Vorrichtung (10) nach einem der vorangegangenen Ansprüche, wobei alle Arbeitsschritte von einer elektronischen zentralen Steuereinheit (30) gesteuert werden.

9. Ein Verfahren zum Spannen einer Sehne vor der Implantation in den Körper, **dadurch gekennzeichnet, dass** es folgende Schritte umfasst:
(a) - Nulleinstellung der Länge der Sehne und Ermitteln des Punktes, an dem die Spannung der Sehne beginnt, zusammen mit einer Erhöhung der angelegten Belastung zuzunehmen;
(b) - Einstellen eines Sollwertes der Maximalbelastung, die an die Sehne angelegt werden soll;
(c) - Erhöhen des Wertes der angelegten Belastung in Richtung des in Schritt (b) eingestellten Maximalwertes, bis ein Absinken der an die Sehne angelegten Zugspannung erkannt wird;
(d) - Ermitteln des Viskositätsüberschneidungspunktes der Gewebekomponenten der Sehne und der Belastung, bei der sich diese Überschneidung vollzieht; und
(e) - Zurückkehren zu einem Belastungswert an der Sehne unterhalb des Belastungswertes, der dem Viskositätspunkt entspricht und an dem die Elastizität der Gewebe wiederhergestellt ist.

10. Verfahren nach Anspruch 9, wobei die Differenz zwischen dem Belastungswert gemäß Schritt (d) und dem Belastungswert gemäß Schritt (e) ermittelt wird.

## Revendications

1. Dispositif de tension (10) permettant de tendre un tendon (25) comprenant :
- une glissière (14) et un élément de soutien (23a), ledit tendon (25) étant retenu par des moyens de retenue (21, 24) formés d'un seul tenant avec ladite glissière (14) et ledit élément de soutien (23a), respectivement ;
- des moyens de détection (19) permettant de détecter la tension réelle induite dans ledit tendon (25) ; le dispositif de tension (10) étant **caractérisé par** :
- un moteur (13) relié mécaniquement à ladite glissière (14) et de manière à déplacer ladite glissière (14) dans deux directions (F1, F2).

2. Dispositif (10) selon la revendication 1, dans lequel ledit moteur (13) est un moteur électrique (13), en particulier un moteur pas à pas électrique (13).

3. Dispositif (10) selon la revendication 2, dans lequel le moteur pas à pas électrique (13) est un moteur électrique à 512 pas/par révolution (13).

4. Dispositif (10) selon l'une quelconque des revendications précédentes, et comprenant également des moyens (27, 29) permettant de déterminer la position de ladite glissière (14) et par conséquent l'étirement dudit tendon (25).

5. Dispositif (10) selon l'une quelconque des revendications précédentes, et comprenant également un écran (34).

6. Dispositif (10) selon la revendication 5, dans lequel l'écran (34) affiche des graphiques montrant, le long de l'axe des x, les valeurs de charge induites dans ledit tendon (25) par déplacement de ladite glissière (14), et, le long de l'axe des y, les valeurs de tension réelles détectées dans le tendon (25) et mesurées par un dynamomètre (19).

7. Dispositif (10) selon la revendication 5, dans lequel des graphiques montrent le moment où le point de viscosité proche de la limite élastique est atteint, et quantifient la différence entre la valeur de charge à laquelle le croisement visqueux des tissus du tendon (25) se produit, et la valeur de charge à laquelle la performance élastique des tissus du tendon (25) est restaurée

8. Dispositif (10) selon l'une quelconque des revendications précédentes, dans lequel toutes les opérations sont commandées par une unité de commande centrale électronique (30).

9. Procédé de tension d'un tendon avant d'être implanté dans le corps, **caractérisé par** les étapes consistant à :
(a) - remettre à zéro la longueur du tendon et à déterminer le point auquel la tension sur le tendon commence à augmenter avec une augmentation de la charge appliquée ;
(b) - fixer une valeur cible de la charge maximum devant être appliquée au tendon ;
(c) - augmenter la valeur de charge appliquée en direction de la valeur maximum fixée à l'étape (b) jusqu'à ce qu'une baisse de la tension appliquée au tendon soit détectée ;
(d) - déterminer le point de croisement visqueux des tissus des composants du tendon, et la charge à laquelle le croisement se produit ; et
(e) - revenir à une valeur de charge, sur le tendon, en-dessous de celle correspondant au point de viscosité, et à laquelle la performance élastique des tissus est restaurée.

10. Procédé selon la revendication 9, dans lequel la différence entre la valeur de charge selon l'étape (d) et la valeur de charge selon l'étape (e) est déterminée.
